# EUROPEAN PATENT SPECIFICATION

(11) **EP 1 864 617 B1**
(45) Date of publication and mention of the grant of the patent: **07.10.2009**
(21) Application number: 07109218.3
(22) Date of filing: 30.05.2007
(51) Int. Cl.: A61B 17/16, A61B 17/17

(54) **Offset tool guide for femoral head preparation**
Offset-Werkzeugführung zur Herstellung eines Femurkopfes
Guide d'outil décalé pour la préparation de la tête de fémur

(30) Priority: 08.06.2006 US 449328
(43) Date of publication of application: 12.12.2007
(73) Proprietor: Howmedica Osteonics Corp., Mahwah, NJ 07430 (US)
(72) Inventor: Tulkis, Peter, Paramus, NJ 07652 (US)
(74) Representative: Le Forestier, Eric

(56) References cited:
- EP-A- 1 502 550
- EP-A- 1 563 795
- WO-A-20/04032767
- US-A- 5 282 803

## Description

### BACKGROUND OF THE INVENTION

The present invention relates to systems, kits and methods for joint replacement requiring preparation of the femoral head. More particularly, the present invention includes tools and methods to correctly position preparation tooling to establish the surgical positioning of an implant for a femoral head.

Artificial joint prostheses are widely used today, restoring joint mobility to patients affected by a variety of conditions, including degeneration of the joint and bone structure. Typically, the failed bone structure is replaced with an orthopedic implant that mimics, as closely as possible, the structure of the natural bone and performs its functions. The satisfactory performance of these implants can be affected not only by the design of the component itself, but also by the surgical positioning of the implanted component and the long-term fixation of the implant. Improper placement or positioning of the implant can adversely affect the goal of satisfactorily restoring the clinical bio-mechanics of the joint as well as impairing adequate fixation of the component when implanted.

Implantable joint prostheses have long been used to provide an artificial hip. When the prosthesis is situated in this position, significant forces such as axial, bending, and rotational forces are imparted to the device. Conventional total hip replacements use an intramedullary stem as part of the femoral prosthesis. The stem passes into the marrow cavity of the femoral shaft. These stem type prostheses are very successful but when they fail the stem can create considerable damage inside the bone. The implant can move about inside the bone causing the intramedullary cavity to be damaged. Because a stiff stem transmits the forces more directly into the femoral shaft, such implants have the further disadvantage that they can weaken the surrounding bone proximal to the hip joint due to stress shielding.

The need often arises to replace at least a portion of a hip implant. Prior art designs often require the entire implant to be replaced even if only a portion of the implant fails. Similarly, the entire implant may have to be replaced if the implant is intact but certain conditions surrounding the implant have changed. This is often due to the implant suffering from a decrease in support from the adjacent bone from stress shielding or other negative effects of the implant on surrounding bone.

Surgeons have sought a more conservative device than an implant using an intramedullary stem as part of the femoral prosthesis. There have been a number of attempts at implants using short stems or femoral caps without stems and requiring less extensive surgery. This type of prosthesis is generally known as a hip resurfacing prosthesis as opposed to a total hip prosthesis. In the mid-1940's Judet in France designed a prosthesis whereby the majority of the femoral head was removed and a replacement device was fitted with a peg or nail which passed a short way down the femoral neck. Small movement of the device against the bone caused friction of the bone and the bending loads on the peg often caused them to break out underneath the bony femoral neck. In the mid-1970's, double cup type arthroplasty was tried. There were several designs: Wagner in Germany, an Italian Group, Imperial College London and the Tharies design from Amstutz in California. These all removed a fair proportion of the femoral bearing surface by turning it down to a cylindrical form or hemispherical form. A metal shell was then fixed with bone cement on the remaining bony peg. The acetabular cup was conventional. Unlike normal total hips, however, which have standard femoral head sizes in the range of 22-32 mm, these double cup arthroplasties needed to have large bearing surface diameters closer to the original hip, typically in a range from 40-50 mm. These latter double cup designs commonly failed either by a crack progressing around the bone cement between the prosthetic femoral shell and the bone or by a fracture of the bone across from one side of the prosthetic femoral component rim to the other.

Current approaches to femoral head resurfacing can be traced back to Amstutz in U.S. Patent 4,123,806. In the '806 patent, a hemispherical cap is cemented to a prepared femoral head while preserving a substantial portion of the femoral head. In U.S. Patent 6,156,069, Amstutz shows a femoral head resurfacing implant having a stem. A similar femoral head resurfacing technique called Birmingham Hip Resurfacing has been developed by McMinn in the United Kingdom. A modular approach to a femoral hip resurfacing is shown in U. S . Patent 4,846,841 to Oh. In this approach, a frustro-conical cap is press-fit to a prepared femoral head. A ball component is then attached to and retained by the cap using a Morse taper fit. A similar approach is shown in U.S. Patent 5,258,033 to Lawes and Ling, which shows a ball component cemented either directly to a prepared head or additionally retained by a press-fit with a frustro-conical cap.

All of these more modern hip resurfacing approaches require that the femoral head be prepared to provide a properly oriented and shaped bone interface for the implant by shaping the head. The outer prepared bone interface with the implant is usually symmetrical around an axis passing through the central region of the femoral neck and is typically cylindrical or conical but may be a more complex solid of revolution. The proximal portion of the prepared head can be a flat surface, tapered, domed, chamfered, or any combination of these features and is usually performed as a separate resection following preparation of the outer interface surface. If a stem is used, it may be cylindrical, tapered or a more complex solid of revolution and is typically short compared to a conventional intramedullary stem. The portion of the bone that hosts the prosthesis must be shaped so that it matches the shape of the prosthesis. The size and shape of the bone may fit exactly the shape and size of the prosthesis or may provide room for cementing to take place or have an excess of bone in a region to allow press-fit fixation, depending on the preferred fixation method.

Because the desired bone shape of the outer implant interface is symmetrical around an axis, a guide wire introduced into the femoral head is typically used to establish the tooling landmark for the various measuring and cutting tools used in the preparation process by providing an axis of revolution. Based on pre-operative planning, the surgeon initially places the guide wire, either freehand or using measurement and guidance tools based on various anatomical reference points on the femur. In order to place the pin, the pin is impacted or inserted in the proximal surface of the femoral head directed toward the greater trochanter and approximately down the mid-lateral axis of the femoral neck. A gauge having an extended stylus that allows measurement of the position of the pin with respect to the neck is then typically used to make a preliminary check of the pin position. By revolving the gauge, the surgeon can evaluate the position of the pin to ensure that the femoral neck will not be undercut when the cutting tool is revolved around the pin. The surgeon also uses the gauge to evaluate the support the prepared femoral head will provide to the implant. If the surgeon is satisfied that the pin position meets these criteria, the guide wire is used to establish the axis of revolution for the shaping cutter or reamer to prepare the head to receive the implant. If a stem cavity is required, a cannulated drill or reamer is centered on the guide pin to create the cavity after creating the outer surface of the prepared head.

However, it often comes to pass that the initial pin position is unsatisfactory. In that instance, the pin position must be offset or shifted. Typically, the new pin is inserted freehand or by adjusting the guidance tools used to position the initial pin. These methods are less than satisfactory because they do not accurately provide a satisfactory reference, either in rotation or translation, to the datum established by the axis of the initial pin. Therefore, there is a need for a tool guide that provides the ability to accurately reposition a new guide pin based on the datum established by the axis of the initial guide pin.

Despite the use of the gauging methods described above, it also happens that the unsatisfactory pin placement is only determined after the preparation cut is made to the final size. This occurs because only after the cut is made is the surgeon able to fully assess the three dimensional positioning of the implant. In this instance, the available surgical options such as removing additional bone stock, adding bone cement or using a different implant type are sub-optimal. Therefore, there is a need for a method of preparing a femoral head that allows evaluation of an initial preparation, provides tools and methods to reposition a guide pin relative to the initial preparation, establishes a new cutting axis and performs a subsequent preparation of the head based on new axis.

### SUMMARY OF THE INVENTION

An object of the present invention is to more accurately position the preparatory reshaping of a femoral head in preparation for a hip resurfacing implant by providing tools and methods to reposition a cutting guide based on a datum established by the axis of an initial cutting guide.

It is a further object of the invention to provide tools and methods to reposition a guide for a femoral head preparation to a selected translational or angular offset or a combination of translational and angular offsets with respect to a datum established by the axis of an initial guide.

It is also a further object of the invention to establish a new cutting axis for a femoral head preparation by offsetting the axis to a selected translational or angular offset or a combination of translational and angular offsets with respect to a datum established by the axis of an initial guide.

Another object of the invention is to provide a kit of tools to reposition a guide for a femoral head preparation to various known offsets with respect to a datum established by the axis of an initial guide.

An additional object of the invention is to provide a surgical method for selection of a tool from a kit of such tools with an appropriate offset to accurately position a guide pin for a femoral head preparation in order to correct a misplaced initial guide.

According to the present invention, a tool for use in a femoral head preparation has a datum establishing surface for establishing an axial datum based on an initial guide position, an offset guide or bearing for guiding a rotary cutter to rotate about and translate along an offset axis and, optionally, an offset bore for positioning a second guide pin at a known offset to the axial datum.

In the preferred embodiment, a cylindrical datum establishing surface of the tool is adapted to cooperate with a matching counterbore in a femoral head that is coaxial with an initial guide position in the femoral head. When the tool is located in the femoral head counterbore, the offset bearing is used to guide a rotary cutter and perform a preparation cut based on an offset axis.

In order to use the tool, an initial guide post centered on the initial pin is used to control the axial location of an over size cutter to initially prepare the femoral head coaxial with the initial pin. Optionally, the initial pin may directly control the initial cutter. The resulting over size configuration of the femoral head is evaluated by the surgeon to ensure that the femoral neck will not be undercut when the cutting tool is revolved around the pin. The surgeon also evaluates the support and positioning the prepared femoral head will provide to the implant. These evaluations may be directly visual or with the assistance of trial implants fitted in position on the prepared surface or with tools providing measurements or visualization relative to the prepared surface. If the initial guide pin is not correctly positioned, the surgeon then determines the extent and orientation of the offset required to establish a new corrected axial location for a subsequent preparation and selects an appropriate guide post. The initial pin and guide post are removed and the selected offset guide post is placed in the counterbore previously created in the femoral head as a datum. The offset guide post is oriented and provides a bearing oriented to the new corrected axis for a final preparation cut using a final size cutter. If desired, the surgeon may use a bore provided by the offset guide post to orient a new guide pin to the new corrected axis. Optionally, the new pin may directly control the final size cutter.

BRIEF DESCRIPTION OF THE DRAWINGS

FIG. 1 is a side view of a femoral head with a guide wire and a counterbore reamer for preparation of the femoral head in accordance with the present invention;

FIG. 2 is a side view of a femoral head with a guide wire and guide post of the present invention;

FIG. 3 is a view as in FIG.2 with an oversize cutter slidingly engaged on the guide post for preliminary preparation of the femoral head in accordance with the present invention;

FIG. 4 is a view as in FIG. 3 with the oversize cutter performing a preliminary preparation of a femoral head in accordance with the present invention;

FIG. 5 is a view as in FIG. 4 with the oversize cutter withdrawn after a preliminary preparation of a femoral head in accordance with the present invention;

FIG. 6 is perspective view of an offset guide post of the present invention;

FIG. 7 is plan view from 7-7 of FIG. 6 of an offset guide post of the present invention;

FIG. 8 is a view of a femoral head with an offset guide post installed in a counterbore preparatory to installing an offset guide wire according to the present invention;

FIG. 9 is a view of a femoral head as in FIG. 8 with an offset guide post being used to install an offset guide wire according to the present invention;

FIG. 10 is plan view of a femoral head from 10-10 0 of FIG. 9 with an offset guide post installed in a counterbore and an offset guide wire according to the present invention;

FIG. 11 is a view as in FIG.9 with a final cutter slidingly engaged on an offset guide post for final preparation of the femoral head in accordance with the present invention:

FIG. 12 is a plan view from 12-12 in FIG. 11 with the final cutter performing a final preparation of a femoral head in accordance with the present invention; and

FIG. 13 is a view as in FIG. 12 showing the final preparation of a femoral head in accordance with the present invention after removing the tooling.

### DETAILED DESCRIPTION

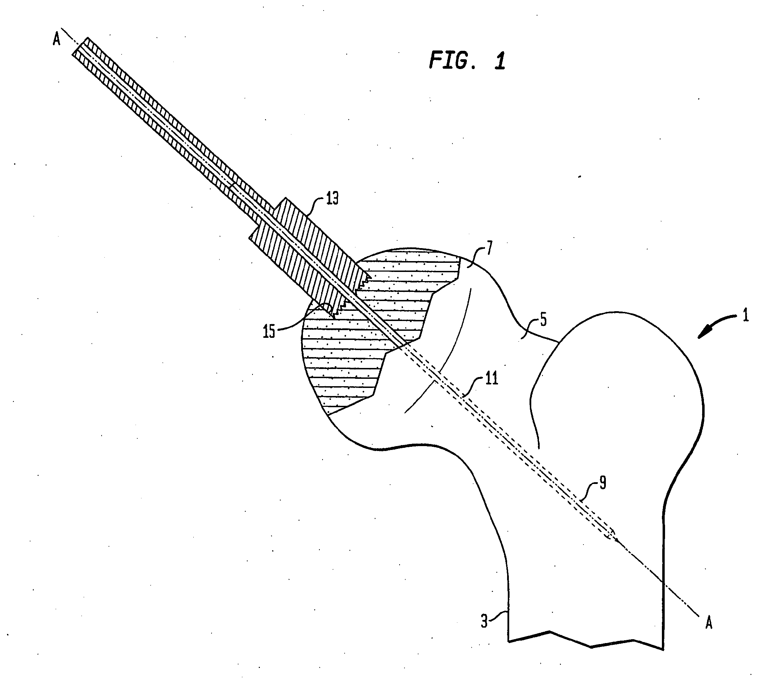

FIGS. 1-13 show an embodiment of the apparatus and method of the present invention to enable an accurately positioned preparation of a femoral head for installation of a hip surfacing implant.

FIG. 1 shows a femur 1 with an upper portion of the proximal femur 3, a femoral neck 5, and a femoral head 7. Based on pre-operative planning, imaging and measurements in situ, an initial guide wire 11, has been placed in the femoral neck 5. The guide wire 11 is impacted or inserted into the femoral head 7 toward the axis of the femoral shaft 3 creating a guide wire bore 9. The guide wire bore 9 and guide wire 11 have a central axis A-A. Coaxial with the axis A-A, the guide wire bore 9 and guide wire 11, is a guide post counterbore 15 located centrally on the femoral head 7. The guide post counterbore 15 is created by the guide post reamer 13. The guide post reamer 13 is cannulated to fit over the guide wire 11 and is capable of rotation about the guide wire. The guide post reamer 13 has cutting features at its distal end to create the guide post counterbore 15 when rotated by suitable tools engaged on a proximal shaft region of the reamer. The guide post reamer 13 is engaged on and guided by the guide wire 11 and brought into contact with the femoral head 7. The reamer 13 is rotated and the guide post counterbore 15 is cut to a predetermined depth. The creation of the counterbore 15 is a preferred embodiment of a step in the method of the invention to establish a datum and to establish axis A-A in subsequent operations as a datum axis for the location of tools to conduct subsequent operations of the invention. The depth of the counterbore serves as a datum to control the depth of the cutter, as will be explained later, should a cutter depth control be desired by the surgeon. While shown as a counterbore in the form of a right circular cylinder, the present invention includes other types of datum location features and corresponding tooling to determine the axis A-A and the distance along axis A-A from a known point in the femoral head as are known in the art. For example, the datum feature could be frustro-conical rather than cylindrical or could be formed by a broach so as to provide surfaces other than a surface of revolution to provide anti-rotation or indexing features.

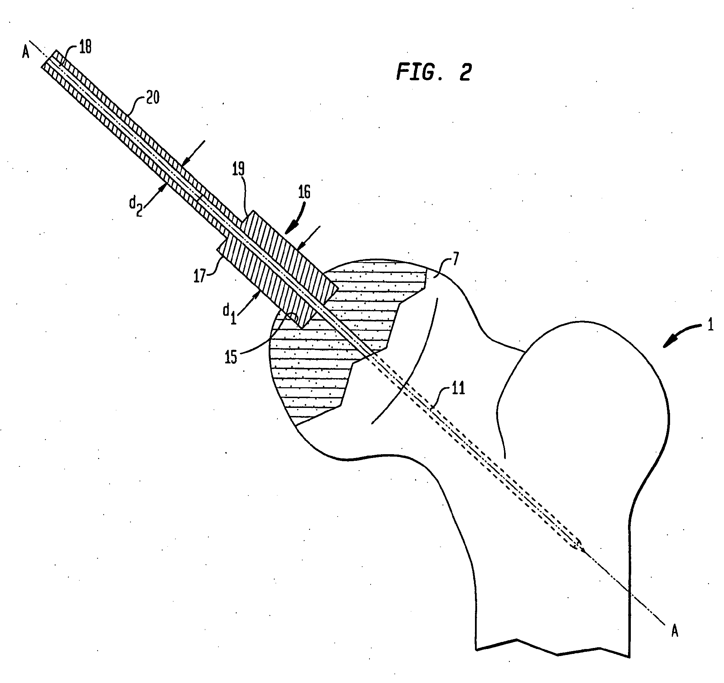

Following creation of the counterbore 15, the reamer 13 is removed, a cannulated guide post 16 is then placed over the guide wire 11, and inserted, as necessary, to engage the guide post 16 with the femoral head 7 by fully seating the guide post 16 in the counterbore 15, as shown in FIG. 2. Guide post 16 has a guide post central bore 18 that closely engages the outer diameter of guide wire 11 and a datum locating cylinder 17 with a major outer diameter d1 that is a slip-fit or slight interference fit in counterbore 15 to insure that guide post 16 is coaxial with guide wire 11 and axis A-A to accurately establish the datum determined by counterbore 15. Also coaxial with axis A-A is a guide post minor diameter d2 extending along the shaft 20 of the guide post 16. The transition between the guide post major diameter d1 and minor diameter d2 is a radial step 19 which will function to limit the travel of a tool slidingly engaged on diameter d2. Of course, should the datum locating features be other than a cylindrical counterbore 15, the datum locating cylinder 17 is suitably reconfigured to locate the appropriate datum features. Optionally, the cutting features of the reamer 13, may be incorporated in the guide post 16. In that instance the reaming operation is performed by the guide post 16 which remains in place after reaming.

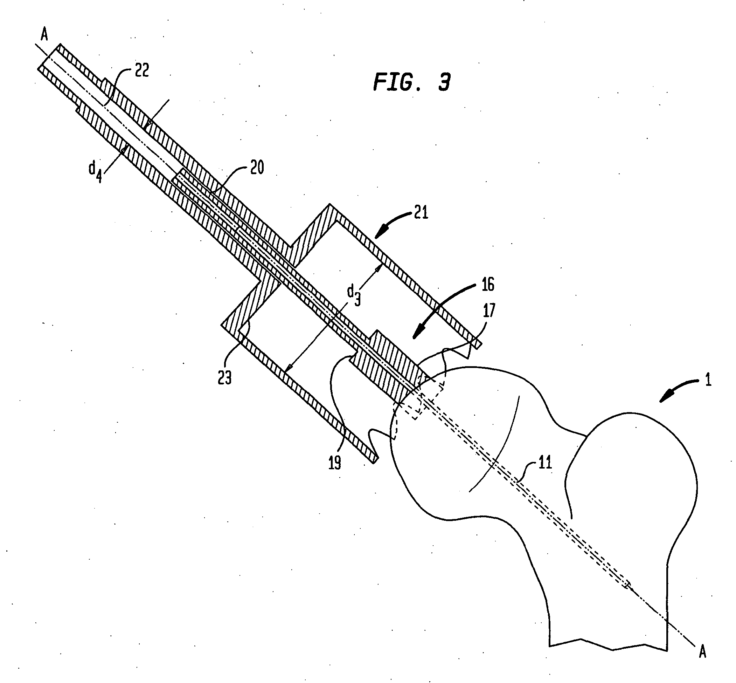

FIG. 3 shows an oversize cutter 21 engaged on the shaft 20 of the guide post 16 in preparation for the initial oversize preparation of the femoral head. In FIG. 3 and subsequent figures, the initial oversize cutter 21, along with a final cutter 41 to be introduced later, are depicted in a configuration for making a cylindrical cut of the femoral head 7. While not shown, other shapes of revolution about an axis, most preferably a cutter of conical shape for producing a frustro-conical cut of the femoral head 7, are also possible. For any shape of cutter, an important aspect of the present invention is that the initial cutter may be oversize to allow assessment of the correct final cutting axis position and provide sufficient excess bone stock to allow a final preparation by offsetting the final cutting axis from the initial axis.

The oversize cutter 21 has a central bore 22 with diameter d4. Diameter d4 is slightly larger than the diameter d2 of the guide post shaft 20 and the cutter 21 is journaled on the shaft 20 and can also translate on the shaft 20. Thus the shaft 20 serves a journal and axial bearing for the cutter 21. The distal or cutting portion of the cutter 21 has cutting features and, in the instance of a cylindrical cutter, an inner diameter d3 that will determine the diameter of the resulting cut of the femoral head 7. The cutter 21 also has a shoulder 23 that provides a planar surface that will engage the cutter stop 19 of the guide post 16 as the cutter translates distally while making the cut. Because the datum locating cylinder 17 is located in the counterbore 15, the guide wire 11, the guide post 16 and the oversize cutter 21 are all coaxial with the axis A-A so that the resulting prepared surface of the femoral head 7 will be a solid of revolution about axis A-A.

Optionally, the guide post 16 may be eliminated and the guide wire 11 is used directly to guide the cutter 21 to rotate and translate about axis A-A. The cutter 21 may be journaled directly on the guide wire 11 with the diameter d4 being reduced to slidingly engage the guide wire.

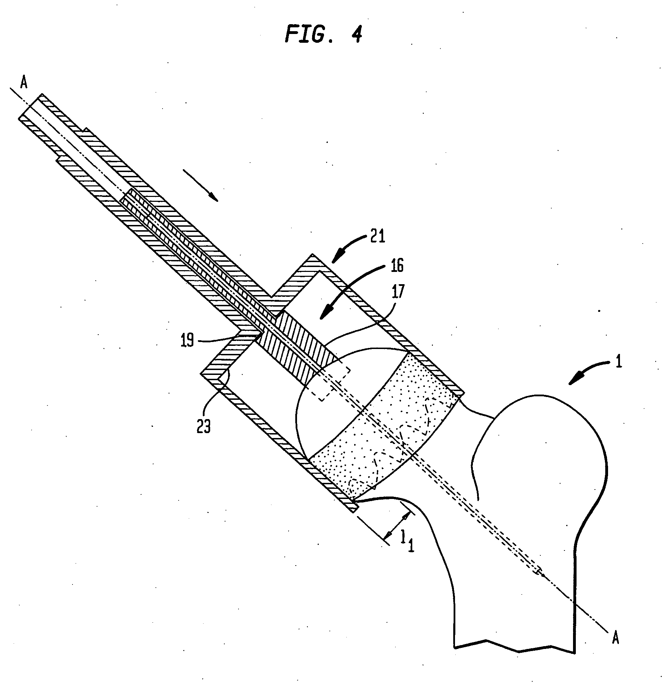

The use of the oversize cutter is depicted in FIG. 4. While rotating the cutter with a suitable driver, the surgeon directs the oversize cutter 21 toward the proximal femur 1 to engage the femoral head 7 and perform the preparation. If control of the depth of the cut is desired, the depth is controlled by the engagement of the cutter upper surface 23 with the cutter stop 19 at a pre-determined height. This height is determined by the depth of the guide post counterbore 15, the length of the guide post 16 from its distal surface to the cutter stop 19 and the length of the cutter 21 from the surface 23 to the distal cutting end. The axial lengths of these components can be available in various lengths to control the depth of cut. The depth of the cut has been previously determined to insure that the head 7 is completely prepared and yet the cutter blade does not contact any undesired portions of the femoral neck 5.

The purpose of the oversize cutter 21 is to create an oversize preparation of the femoral head 7 to allow visual confirmation and physical measurements to determine if the axis A-A is the proper axis for a final preparation or if adjustment of the cutting axis is necessary. An oversize cutter 21 with a sufficient inner diameter d3 is selected by the surgeon to ensure that sufficient bone stock is allowed to offset or reposition the final preparation cutting axis and final cut if necessary.

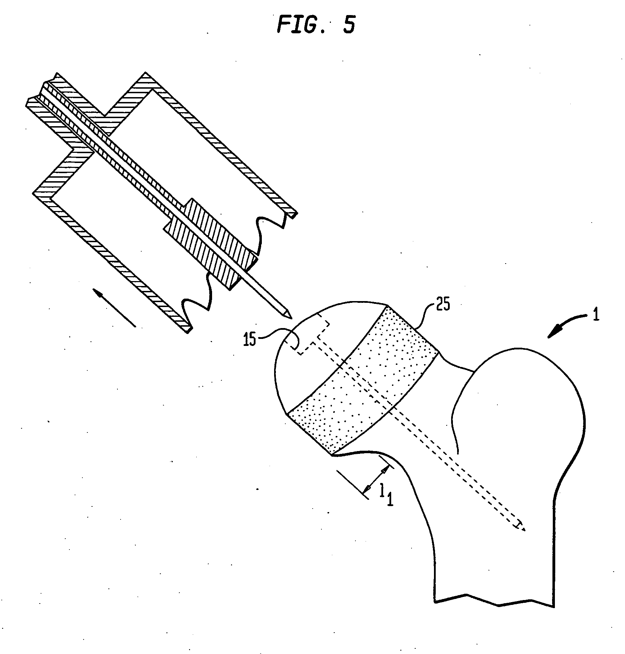

Turning to FIG. 5, the oversize cutter 21, guide post 16, and guide wire 11 have been removed from the now prepared femoral head. The femoral head 7 now has an oversize prepared surface 25. If visual inspection and measurements indicate that the oversize prepared surface 25 is properly positioned with respect to the femoral neck and anatomical considerations, the surgeon can proceed to reinstall the guide wire 11 along with the guide post 16. The surgeon can then use a final size cutter to complete the preparation of this portion of the femoral head.

However, as previously mentioned, it is often the case that the initial pin position and subsequent oversize prepared surface 25 is not in the optimum position. An example is seen in FIG. 5 where the prepared surface 25 is centered too low on the femoral head 7 with the result that the surface creates an overhang of the neck designated by a distance 11. Because the initial cutter 21 was oversize, it is possible to correct this problem by reducing the diameter of the cutter and offsetting a guide post to create a new axis of revolution of the cutter. While the actual selection of the final cutter diameter and the degree of offset is a matter of the surgeon's discretion, it will be seen that in this instance, a new cutter diameter that is nominally of a diameter equal to d3 minus l1, and a translation of the guide post axis upward and parallel to the original axis A-A by a distance of l1 divided by 2, will result in a prepared surface that will preserve a maximum of bone structure, yet eliminate the undesired overhang of the head designated by the dimension l1.

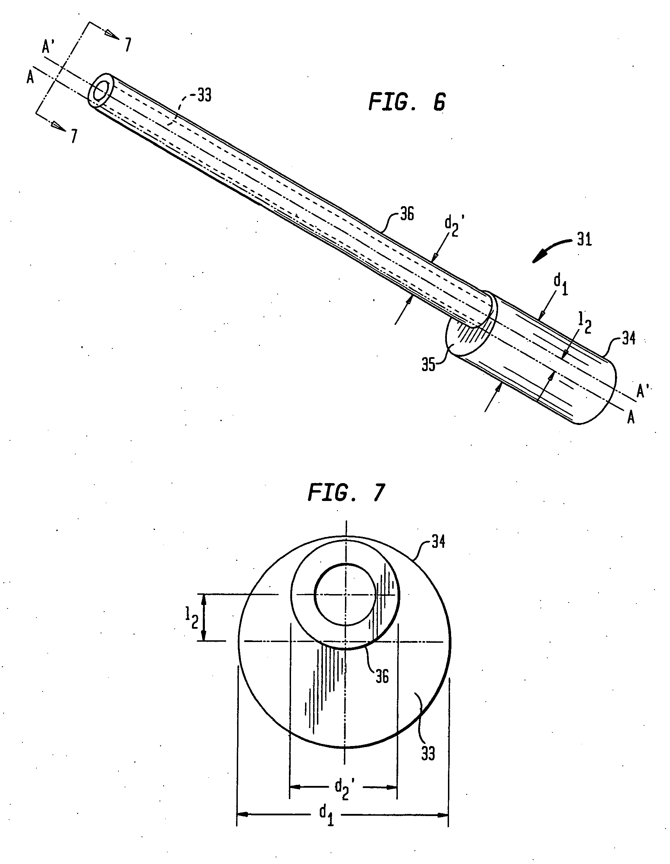

FIG. 6 shows a tool according to an embodiment of the present invention to allow the offset placement of a secondary guide wire and to function as an offset guide post for a final cutter to provide correction of an initial placement of a guide wire that is in a less than optimal position. This tool is an offset guide post 31. Similar to guide post 16, it has a datum locating cylinder 34 with a major diameter d1 sized to fit in the guide post counterbore 15 in the femoral head 7 to mount the guide post 31 on the head and re-establish the axis A-A based on the counterbore datum. When in place on the head, the cylinder 34 is coaxial with the original axis A-A. The offset guide post 31 has an offset bore 33 having an inner diameter of the bore that allows passage of a new guide wire 37. The center line of this bore is offset from the center line A-A by a distance 12 and designated by axis A'-A'. Coaxial with axis A'-A', is an extended bearing or tool guiding surface 36 having diameter d2' on the proximal tool guiding portion of the offset guide post 31. As shown, an offset guide cutter stop 35 is provided by the transition from diameter d1 to diameter d2'. FIG. 7 shows a plan view of the offset guide post shown in FIG. 6.

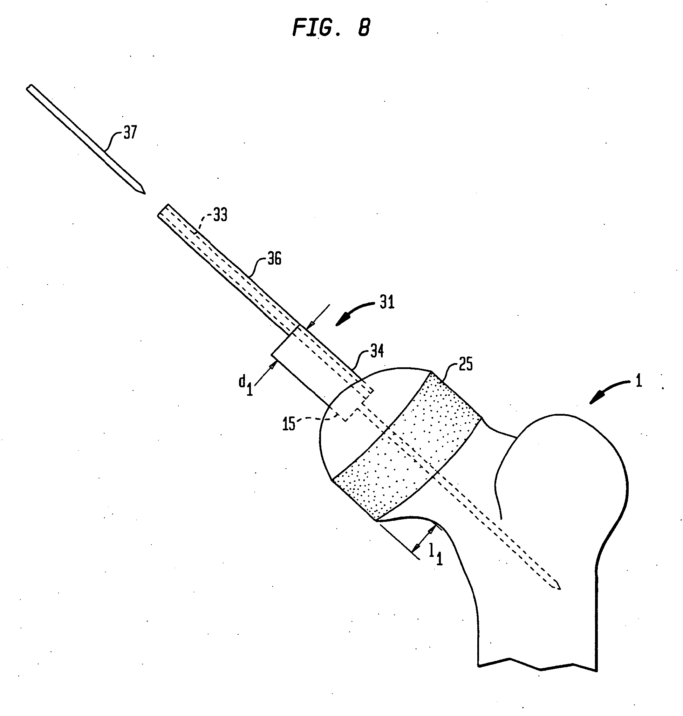

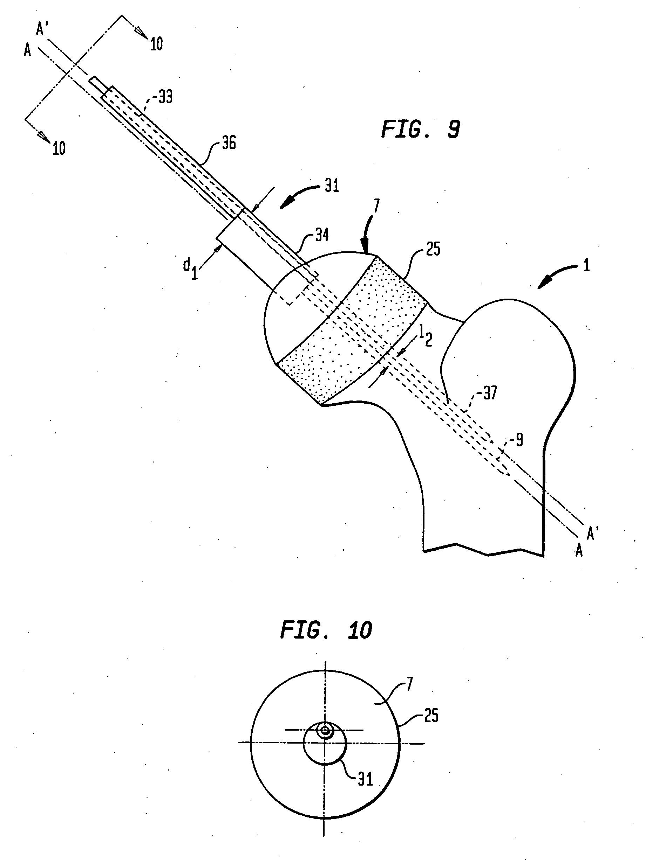

FIG. 8 shows the offset guide post 31 mounted on a femoral head 7 by impacting the distal end defined by diameter d1 into the guide post counterbore 15. As previously noted, the datum locating cylinder 34 is now coaxial with the original axis A-A and the distal end of the offset guide post 31 engages the bottom of the counterbore 15 to establish a depth datum to allow control of the depth of the cut. During placement of the offset guide post 31, the surgeon rotationally orients the bearing or guide portion 36 about axis A-A to the desired angular orientation to achieve the proper direction of offset of the final preparation. In this instance, the guide 36 is positioned to offset the guiding section and consequently the cutter in the upward direction. Having achieved the proper angular orientation, a second guide wire 37 may be inserted through the offset bore 33 as shown in FIG. 9 and 10 if desired. This guide wire 37 provides assurance that the offset guide post does not rotate and also provides a tool for defining the final preparation axis in additional surgical steps such as preparing the proximal portion of the femoral head and fitting the implant to the prepared femoral head. Optionally, the tool guiding surface 36 may be eliminated and the guide wire 37 may directly journal the final size cutter.

For a small offset, the location of the second guide wire 37 may overlap the existing guide wire bore 9, creating the possibility of play in the position of the guide wire when the offset guide post 31 is later removed. In this instance, the stability of the second guide wire 37 can be enhanced by making the diameter of the second guide wire 37 larger than the diameter of the guide wire bore 9 to create a suitable engagement with the bone and prevent movement of the pin.

Using the offset guide post 31 located on the datum established by the guide post counterbore 15, the surgeon may create a controlled offset of the tool guiding portion 36 provided by the minor diameter d2' of the offset guide post 31 in a desired direction. While in the example depicted in FIGS. 1-13 only a single offset of a translation 12 is depicted to create the new axis A'-A', it will be understood that the translation 12 may be any of a number of discrete offset distances. The tool guiding portion 36 may also incorporate an angle of rotation or angular offset such that axis A'-A' may not be parallel to axis A-A and the axes either intersect or are skew. For convenience while performing surgeries, a kit of such tools 31 may be provided for the surgeon with different translational and angular offsets or combinations thereof to allow the surgeon flexibility in determining the translational and angular positioning of a final preparation cut.

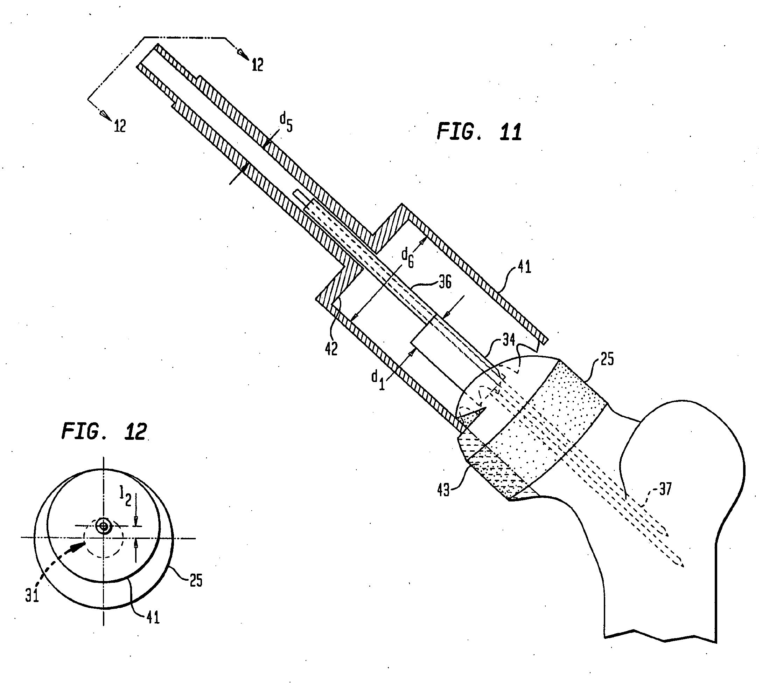

FIGS. 11 and 12 show a final cutter 41 journaled on the guide 36 of the offset guide post 31 in preparation for making a final preparation cut on the femoral head 7. As previously discussed, the diameter of the final cutter d6 is smaller than the diameter d3 of the oversize cutter 21 as determined by the surgeon to remove desired areas of excess bone. In this example, the diameter d6 has been reduced by the distance l1 from the previous diameter d3. In conjunction with the offset of the offset guide post by distance 11 divided by 2 or 12, it can be seen that little or no material will be cut from the upper portion of the femoral head 7, while a relatively large amount of material designated in the prior figures by l1 will be removed from the cross-hatched area designated 43.

The operation of making a preparation with the final cutter 41 proceeds similarly to the operation described for the oversize cutter 21. The final cutter 41 has a central bore with diameter d5 journaled on the bearing or guide 36 and has an upper surface 42 that engages with the offset guide cutter stop 35 to determine the depth of cutting as the cutter 41 is rotated and translated distally along axis A'-A' to make the final cut. Similar to the depth control features described for the initial preparation, guide post 16, the length from the distal end of the guide post 31 to the offset guide cutter stop 35 and the length from the cutter upper surface 42 to the distal end of the cutter can be varied to determine the depth of the cut to insure that a complete preparation is made and that the cutter does not travel too far in the distal direction and undercut the neck.

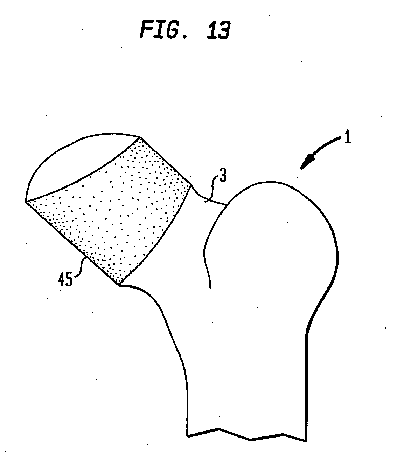

Following the completion of the final preparation cut, the final cutter 41, the offset guide post 31 and the guide wire 37 are removed from the proximal femur as shown in FIG. 13. The resulting final preparation of the femoral head is symmetrical about axis A'-A'. The prepared femoral head is now ready for additional steps required for installing a femoral head resurfacing implant. If desired, the guide wire 37 may be left in place or reinserted to serve as a tooling point for additional steps needing a guide wire. It will be understood that the various terms designated "final", such as final preparation, are relative to the initial terms, such as initial preparation, and do not preclude subsequent preparations using the tools and methods of the invention or other types of preparations to further shape the femoral head.

Of course any combination of the above mentioned embodiments is contemplated by the present invention.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. A surgical offset guide tool (31) for making a subsequent preparation of a natural femoral head by guiding a rotary cutter (41) about and along an offset axis of revolution (A'-A'), the axis being offset from a datum (15) having a datum axis (A-A) coaxial with an initial axis of revolution used to create an initial preparation of the femoral head, comprising:
a mount (31), the mount having a datum locating cylinder (34) and a tool guiding cylinder (36) attached to the datum locating cylinder, the mount also having a mount axis that is coaxial with said datum axis;
an offset axis (A'-A') offset from said mount axis, the offset axis being central axis of the tool guiding cylinder ; and
the tool guiding being capable of guiding said rotary cutter to rotate about said offset axis and translate along said offset axis to produce an external cylindrical surface (45) on the femoral head.

2. The offset guide tool as set forth in claim 1 wherein said tool has a through bore coaxial (33) with said offset axis.

3. The offset guide tool as set forth in claim 1 wherein said mount is a cylinder having a first diameter (d1) .

4. The offset guide tool as set forth in claim 3 wherein said guide is a cylindrical journal having a second diameter (d2'), said journal being coaxial with said offset axis.

5. The offset guide tool as set forth in claim 4 wherein said mount and said journal are adjacent and said first diameter is larger than said second diameter.

6. The offset guide tool as set forth in claim 1 wherein said mount axis is parallel with said offset axis.

7. The offset guide tool as set forth in claim 1 wherein said mount axis and said offset axis are skew axes.

8. The offset guide tool as set forth in claim 7wherein said mount axis and said offset axis intersect.

9. The offset guide tool as set forth in claim 1 wherein said offset guide tool has a stop (35) for limiting the translational guiding along said offset axis to a known axial position relative to said mount.

10. A kit of surgical tools for making an initial and a subsequent preparation of a natural femoral head, the subsequent preparation being made by guiding a rotary cutter about and along an offset axis of revolution, the axis being offset from a datum having a datum axis coaxial with an initial axis of revolution used to create an initial preparation of the femoral head, comprising:
a first guide wire for impaction in a femoral head, having a guide wire axis coaxial with said initial axis of revolution;
a reamer for reaming a counterbore in a femoral head coaxial with said guide wire axis to define said datum;
an offset guide tool according to any one of the claims 1 to 9 ;
an initial rotary cutter adapted to rotate about said initial axis of revolution and having cutting surfaces configured to create an initial preparation shape for a femoral head; and
a subsequent rotary cutter adapted to rotate about said guide of said offset guide tool and having cutting surfaces configured to create a subsequent preparation shape for a femoral head.

11. The kit as set forth in claim 10 wherein said initial preparation shape of said initial rotary cutter is oversize with respect to said subsequent preparation shape of said subsequent rotary cutter.

12. The kit as set forth in claim 10 further comprising a guide tool having a mount with a mount axis for mounting said tool in a known position relative to said datum and said datum axis such that said mount axis is coaxial with said datum axis, and a guide capable of guiding said initial rotary cutter to rotate about said mount axis and translate along said mount axis.

## Patentansprüche

1. Chirurgisches Instrument zur Offset-Führung (31), um eine Nachpräparation eines natürlichen Hüftkopfes vorzunehmen, indem eine rotierende Schneidvorrichtung (41) um eine Offset-Drehachse (A'-A') herum und daran entlang geführt wird, wobei die Achse im Verhältnis zu einem Bezugspunkt (15) versetzt ist, der eine Bezugsachse (A-A) hat, die zu einer Erstdrehachse koaxial ist, die verwendet wird, um eine Erstpräparation des Hüftkopfes zu erstellen, umfassend:
eine Halterung (31), wobei die Halterung einen Bezugspunkt-Festlegungszylinder (34) und einen Instrumenten-Führungszylinder (36), der an dem Bezugspunkt-Festlegungszylinder angebracht ist, aufweist, wobei die Halterung auch eine Halterungsachse aufweist, die zur Bezugsachse koaxial ist;
eine Offset-Achse (A'-A'), die im Verhältnis zur Halterungsachse versetzt ist, wobei die Offset-Achse eine Mittelachse des Instrumenten-Führungszylinders ist; und
wobei die Instrumentenführung in der Lage ist, die rotierende Schneidvorrichtung zu führen, damit sich diese um die Offset-Achse dreht und sich an der Offset-Achse entlang verschiebt, um eine externe zylindrische Oberfläche (45) an dem Hüftkopf hervorzubringen.

2. Offset-Führungsinstrument nach Anspruch 1, wobei das Instrument eine durchgehende Bohrung (33) aufweist, die zur Offset-Achse koaxial ist.

3. Offset-Führungsinstrument nach Anspruch 1, wobei die Halterung ein Zylinder ist, der einen ersten Durchmesser (d1) aufweist.

4. Offset-Führungsinstrument nach Anspruch 3, wobei die Führung ein zylindrischer Lagerzapfen ist, der einen zweiten Durchmesser (d2') aufweist, wobei der Lagerzapfen zur Offset-Achse koaxial ist.

5. Offset-Führungsinstrument nach Anspruch 4, wobei die Halterung und der Lagerzapfen nebeneinander liegen und der erste Durchmesser größer ist als der zweite Durchmesser.

6. Offset-Führungsinstrument nach Anspruch 1, wobei die Halterungsachse zur Offset-Achse parallel ist.

7. Offset-Führungsinstrument nach Anspruch 1, wobei die Halterungsachse und die Offset-Achse Schrägachsen sind.

8. Offset-Führungsinstrument nach Anspruch 7, wobei sich die Halterungsachse und die Offset-Achse schneiden.

9. Offset-Führungsinstrument nach Anspruch 1, wobei das Offset-Führungsinstrument einen Anschlag (35) aufweist, um die Translationsführung an der Offset-Achse entlang auf eine mit Bezug auf die Halterung bekannte Axialposition zu begrenzen.

10. Chirurgischer Instrumentensatz zum Ausführen einer Erstpräparation und einer Nachpräparation eines natürlichen Hüftkopfes, wobei die Nachpräparation ausgeführt wird, indem eine rotierende Schneidvorrichtung um eine Offset-Drehachse herum und daran entlang geführt wird, wobei die Achse im Verhältnis zu einem Bezugspunkt versetzt ist, der eine Bezugsachse aufweist, die zu einer Erstdrehachse koaxial ist, die verwendet wird, um eine Erstpräparation des Hüftkopfes zu erstellen, umfassend:
einen ersten Führungsdraht zur Impaktierung in einem Hüftkopf, der eine Führungsdrahtachse aufweist, die zur Erstdrehachse koaxial ist;
eine Reibahle zum Aufreiben einer Senkung in einem Hüftkopf, die zur Führungsdrahtachse koaxial ist, um den Bezugspunkt zu definieren;
ein Offset-Führungsinstrument nach einem der Ansprüche 1 bis 9 ;
eine rotierende Erstschneidvorrichtung, die dazu geeignet ist, um sich um die Erstdrehachse zu drehen, und Schneidflächen aufweist, die ausgestaltet sind, um eine Erstpräparationsform für einen Hüftkopf zu erstellen; und
eine rotierende Nachschneidvorrichtung, die dazu geeignet ist, um sich um die Führung des Offset-Führungsinstruments zu drehen, und Schneidflächen aufweist, die ausgestaltet sind, um eine Nachpräparationsform für einen Hüftkopf zu erstellen.

11. Satz nach Anspruch 10, wobei die Erstpräparationsform der rotierenden Erstschneidvorrichtung im Verhältnis zu der Nachpräparationsform der rotierenden Nachschneidvorrichtung überdimensioniert ist.

12. Satz nach Anspruch 10, ferner umfassend ein Führungsinstrument mit einer Halterung mit einer Halterungsachse, um das Instrument in einer im Verhältnis zum Bezugspunkt und der Bezugsachse bekannten Position zu haltern, so dass die Halterungsachse zur Bezugsachse koaxial ist, und eine Führung, die in der Lage ist, das rotierende Erstschneidwerkzeug zu führen, damit es sich um die Halterungsachse dreht und sich an der Halterungsachse entlang verschiebt.

## Revendications

1. Instrument chirurgical de guidage à décalage (31) pour réaliser une préparation postérieure d'une tête de fémur naturelle en guidant un outil de coupe rotatif (41) autour et le long d'un axe de rotation à décalage (A'-A'), l'axe étant décalé par rapport à une référence (15) ayant un axe de référence (A-A) coaxial à un axe de rotation initial utilisé pour créer une préparation initiale de la tête de fémur, comprenant :
une monture (31), la monture ayant un cylindre de localisation de référence (34) et un cylindre de guidage d'instrument (36) attaché au cylindre de localisation de référence, la monture ayant également un axe de monture qui est coaxial audit axe de référence ;
un axe à décalage (A'-A') décalé par rapport audit axe de monture, l'axe à décalage étant un axe central du cylindre de guidage d'instrument ; et
le guide d'instrument étant capable de guider ledit outil de coupe rotatif pour qu'il tourne autour dudit axe à décalage et se translate le long dudit axe à décalage pour produire une surface cylindrique externe (45) sur la tête de fémur.

2. Instrument de guidage à décalage selon la revendication 1, dans lequel ledit instrument présente un alésage traversant (33) coaxial audit axe à décalage.

3. Instrument de guidage à décalage selon la revendication 1, dans lequel ladite monture est un cylindre ayant un premier diamètre (d1) .

4. Instrument de guidage à décalage selon la revendication 3, dans lequel ledit guide est un tourillon cylindrique ayant un deuxième diamètre (d2'), ledit tourillon coaxial audit axe à décalage.

5. Instrument de guidage à décalage selon la revendication 4, dans lequel ladite monture et ledit tourillon sont adjacents et ledit premier diamètre et plus grand que ledit deuxième diamètre.

6. Instrument de guidage à décalage selon la revendication 1, dans lequel ledit axe de monture est parallèle audit axe à décalage.

7. Instrument de guidage à décalage selon la revendication 1, dans lequel ledit axe de monture et ledit axe à décalage sont des axes en biais.

8. Instrument de guidage à décalage selon la revendication 7, dans lequel ledit axe de monture et ledit axe à décalage se coupent.

9. Instrument de guidage à décalage selon la revendication 1, dans lequel ledit instrument de guidage à décalage présente une butée (35) pour limiter le guidage en translation le long dudit axe à décalage à une position axiale connue par rapport à ladite monture.

10. Kit d'instruments chirurgicaux pour réaliser une préparation initiale et une préparation postérieure d'une tête de fémur naturelle, la préparation postérieure étant réalisée en guidant un outil de coupe rotatif autour et le long d'un axe de rotation à décalage, ledit axe étant décalé par rapport à une référence ayant un axe de référence coaxial à un axe de rotation initial utilisé pour créer une préparation initiale de la tête de fémur, comprenant :
un premier fil de guidage pour l'impaction dans une tête de fémur, ayant un axe de fil de guidage, coaxial audit axe de rotation initial ;
un alésoir pour aléser un suralésage dans une tête de fémur, coaxial audit axe de fil de guidage pour définir ladite référence ;
un instrument de guidage à décalage selon l'une quelconque des revendications 1 à 9 ;
un outil de coupe rotatif initial adapté pour tourner autour dudit axe de rotation initial et ayant des surfaces de coupe configurées pour créer une forme de préparation initiale pour une tête de fémur ; et
un outil de coupe rotatif postérieur adapté pour tourner autour dudit guide dudit instrument de guidage à décalage et ayant des surfaces de coupe configurées pour créer une forme de préparation postérieure pour une tête de fémur.

11. Kit selon la revendication 10, dans lequel la forme de préparation initiale dudit outil de coupe rotatif initial est surdimensionnée par rapport à ladite forme de préparation postérieure dudit outil de coupe rotatif postérieur.

12. Kit selon la revendication 10, comprenant en outre un instrument de guidage ayant une monture avec un axe de monture pour monter ledit instrument dans une position connue par rapport à ladite référence et audit axe de référence, et un guide capable de guider ledit outil de coupe rotatif pour tourner autour dudit axe de monture et se déplacer en translation le long dudit axe de monture.
